# EUROPÄISCHE PATENTSCHRIFT

(11) **EP 4 568 598 B1**
(45) Veröffentlichungstag und Bekanntmachung des Hinweises auf die Patenterteilung: **27.05.2026**
(21) Anmeldenummer: 23739203.0
(22) Anmeldetag: 04.07.2023
(51) Int. Cl.: A61B 17/66

(54) **FIXIERVORRICHTUNG ZUR TRANSVERSALEN KNOCHENDISTRAKTION**
FIXING DEVICE FOR TRANSVERSE BONE DISTRACTION
DISPOSITIF DE FIXATION POUR DISTRACTION OSSEUSE TRANSVERSALE

(30) Priorität: 10.08.2022 DE 102022120185
(43) Veröffentlichungstag der Anmeldung: 18.06.2025
(73) Patentinhaber: Stauch, Roman, 97959 Assamstadt (DE)
(72) Erfinder: Stauch, Roman, 97959 Assamstadt (DE)
(74) Vertreter: Caspary, Karsten
(86) Internationale Anmeldenummer: PCT/EP2023/068453
(87) Internationale Veröffentlichungsnummer: WO 2024/032985

(56) Entgegenhaltungen:
- EP-A1- 3 410 964
- EP-B1- 3 410 964
- CN-A- 105 167 828
- CN-A- 106 108 992
- CN-U- 203 183 002

## Beschreibung

Die vorliegende Patentanmeldung betrifft eine Fixiervorrichtung zur transversalen Knochendistraktion.

Derartige Vorrichtungen werden auf dem Gebiet der Orthopädie bzw. der Extremitätenkorrektur insbesondere, aber nicht nur bei Patienten eingesetzt, die an Diabetes erkrankt sind. Nach Angaben der US-Gesundheitsbehörde CDC waren im Jahre 2007 etwa 7,8 % der gesamten US-Bevölkerung an Diabetes erkrankt, was fast 24 Millionen Menschen entspricht. Bei diesen Patienten tritt häufig die Problematik auf, dass besonders der Bereich der Unterschenkel unzureichend durchblutet ist, was in Kombination mit einer diabetischen Vorerkrankung bei Verletzungen in diesem Bereich zu einem dramatischen Verlauf bis hin zur Amputation führen kann. Die Behandlung dieser Patienten und die dabei oft auftretenden Komplikationen verursachten beispielsweise in den USA im Jahre 2007 indirekte und direkte Gesamtkosten in Höhe von 174 Milliarden US-Dollar.

Periphere vaskuläre Komplikationen und neurologische Komplikationen, die eng mit Fußulzerationen verbunden sind, machten dabei 31 % bzw. 24 % der Kosten aus und gehörten zu den Hauptverursachern der stationären Aufenthaltsdauer. Mehr als 60 % der nichttraumatischen Amputationen der unteren Gliedmaßen treten bei Diabetikern auf, und mindestens 80 % der Amputationen geht ein Ulkus voraus.

In den vergangenen Jahren wurde festgestellt, dass durch eine Querdistraktion bzw. eine transversale Distraktion mittels einer externen Fixiervorrichtung die Durchblutung der unteren Extremität deutlich verbessert und dadurch eine Amputation in aller Regel verhindert werden kann. In mehreren Studien, unter anderem in China, wurde dieser Sachverhalt bestätigt, beispielsweise wurden dort bereits mehr als eintausend Patienten mit einer derartigen externen Fixiervorrichtung bei einer transversalen Knochendistraktion behandelt. Dabei wird ein Teil des Unterschenkelknochens (Tibia) herausgetrennt und durch Querdistraktion dieses Teils neues Knochengewebe generiert.

Die chinesischen Offenlegungsschriften CN 106 108 992 A und CN 105 167 828 A offenbaren eine gattungsgemäße Fixiervorrichtung zur transversalen Knochendistraktion mit einem Hauptkörper und einem parallel dazu angeordneten Nebenkörper, an deren Enden jeweils Aufnahmeeinrichtungen für Knochenstifte bzw. Kirschner-Drähte angeordnet sind. Hauptkörper und Nebenkörper sind über einen Schraubmechanismus miteinander verbunden, wobei eine im Hauptkörper angeordnete Rändelmutter eine Gewindestange bewegen kann, deren knochenzugewandtes Ende mit dem Nebenkörper in dessen Mitte verbunden ist.

Die CN 203 183 002 U beschreibt ebenfalls eine Fixiervorrichtung zur Knochendistraktion mit Kirschner-Drähten, bei der der in Querrichtung verstellbare Nebenkörper auf der knochenabgewandten Seite des Hauptkörpers angeordnet ist.

Diese bekannten Vorrichtungen weisen jedoch einige Nachteile auf. Insbesondere führen der Aufbau und die Struktur mit den zahlreichen Verstellmöglichkeiten dazu, dass die Handhabung für den Operateur sehr aufwendig und nur unter hohem Zeitaufwand allein durchführbar ist. Diese große Anzahl an Verstelloptionen ist im Operationssaal nicht notwendig und erschwert lediglich den Umgang mit der Vorrichtung, wodurch sich die Operationsdauer unnötig verlängern kann. Des Weiteren besteht durch die Geometrie der bekannten Vorrichtung mit den langen überstehenden Stiften und Schrauben die Gefahr, dass der Patient mit der an seinem Unterschenkel befestigten Vorrichtung an einem Tisch- oder Stuhlbein oder anderweitig hängenbleibt. Des Weiteren ist die Struktur beim täglichen Gebrauch nachteilig, insbesondere beim Anziehen von Kleidung oder während des Schlafens.

Die EP 3 410 964 A0 offenbart eine Fixiervorrichtung zur longitudinalen Knochendistraktion.

Es ist daher die Aufgabe der vorliegenden Erfindung, die Nachteile des Standes der Technik zumindest teilweise zu überwinden und eine Fixiervorrichtung bereitzustellen, die problemlos handhabbar, einfach aufgebaut und leicht ist sowie den täglichen Gebrauch und die Verwendung während einer Operation erleichtert.

Diese Aufgabe wird durch den Gegenstand des Anspruchs 1 gelöst. Vorteilhafte Ausgestaltungen und Ausführungsformen sind in den abhängigen Ansprüchen beschrieben.

Erfindungsgemäß wird eine Fixiervorrichtung zur transversalen Knochendistraktion bereitgestellt mit einem länglichen Grundkörper, der eine knochenabgewandte Oberseite, eine knochenzugewandte Unterseite, einen Mittelabschnitt sowie ein erstes Ende und ein zweites Ende aufweist, einer ersten äußeren Schanzschraube, die an dem ersten Ende auf der Unterseite des Grundkörpers angebracht ist, und einer zweiten äußeren Schanzschraube, an dem zweiten Ende auf der Unterseite des Grundkörpers angebracht ist, mindestens einer inneren Schanzschraube, die in dem Mittelabschnitt auf der Unterseite des Grundkörpers angebracht ist, und mindestens einer Verschiebeeinrichtung, die dazu eingerichtet ist, die mindestens eine innere Schanzschraube relativ zu den äußeren Schanzschrauben vom Knochen weg zu verschieben, wobei die inneren und äußeren Schanzschrauben dazu eingerichtet sind, an ihrem knochenzugewandten Ende mit dem Knochen in Eingriff gebracht zu werden, wobei die mindestens eine innere Schanzschraube, die äußeren Schanzschrauben und die Verschiebeeinrichtung in einem funktionalen Zustand der Fixiervorrichtung nicht über eine knochenabgewandte Oberfläche der Fixiervorrichtung hinausragen, so dass die knochenabgewandte Oberfläche der Fixiervorrichtung eine im Wesentlichen ebene, vorsprungsfreie Fläche bildet.

Durch die ebene Oberfläche der Fixiervorrichtung ohne Vorsprünge und Spitzen bzw. Kanten wird eine klar verbesserte Handhabung und eine geringere Verletzungsgefahr für den Patienten erreicht. Darüber hinaus erleichtert der verhältnismäßig einfache Aufbau die Bedienung während der Operation. Zudem ergibt sich durch die Struktur eine geringe Bauhöhe und ein geringes Gewicht der Vorrichtung, weil dadurch auch Länge und Breite ebenfalls reduziert werden können. Unter einer im Wesentlichen ebenen Oberfläche der Fixiervorrichtung ist hier keine durchgängige, glatte Fläche zu verstehen; vielmehr zählen auch andere Bestandteile zu der Oberfläche wie z. B. die Verschiebeeinrichtung oder andere, in die ebene Oberfläche integrierten Elemente, die die Handhabung nicht durch Vorsprünge, Kanten, Spitzen oder Schrauben bzw. Stifte usw. verschlechtern würden. Entscheidend ist, dass die Übergänge zwischen den Bestandteilen, z. B. zwischen der Oberseite des Grundkörpers und der Verschiebeeinrichtung derart ausgebildet sind, dass sich Kabel, Schläuche, (Kleidungs-) Stoffe und dergleichen nicht daran verhaken oder verheddern können. Für die Bestandteile an den Übergängen gilt demnach, dass sie entsprechend abgerundet, abgeschrägt oder abgeflacht sein können. Des Weiteren lassen sich durch die flexible Anordnung der Verschiebeeinrichtung einfach Konstruktionen erzielen, die Bauraum, zusätzliche Elemente und damit Kosten einsparen und einen vereinfachten Aufbau ermöglichen.

Dabei können in bevorzugten Ausführungsformen bei Betätigung der Verschiebeeinrichtung a) die mindestens eine innere Schanzschraube mit dem Grundkörper festgelegt sein, oder b) die mindestens eine innere Schanzschraube relativ zum Grundkörper bewegbar sein. Bei Ausführungsform a) ergibt sich eine besonders einfache, schlanke Konstruktion, weil auf zusätzliche Elemente verzichtet werden kann. Für den Operateur bzw. das medizinische Personal ist daher die Handhabung vereinfacht. Bei Ausführungsform b) ist insbesondere eine genaue Einstellung des Maßes der Verschiebung durch die Verschiebeeinrichtung möglich, weil die Verschiebeeinrichtung nur mit einem Element verstellt wird.

In einer vorteilhaften Ausführungsform weist die Verschiebeeinrichtung mindestens ein Teil aus der Gruppe auf, die einen Rändel- oder Drehkopf, eine Rändelmutter, eine Rändelschraube, eine Schraube, einen profilierten Schraubenkopf und ein Führungselement umfasst. Auch andere Ausbildungen der Verschiebeeinrichtung, die leicht mit der Hand bedient werden können, sind möglich. Damit soll ein Verstellelement der Verschiebeeinrichtung die Relativverschiebung von mindestens einer Schanzschraube bezogen auf den Grundkörper bewirken. Verstellelemente mit Rändelprofil sind einfach strukturiert, günstig verfügbar und in der Funktion zuverlässig. Sie können beispielsweise aus einem geeigneten Metall bzw. Metalllegierung oder einem geeigneten Kunststoff wie beispielsweise Polypropylen (PP), Polycarbonat (PC), Acrylnitril-Butadien-Styrol (ABS), Polyetheretherketon (PEEK) oder einem anderen geeigneten festen Polymerwerkstoff ausgebildet sein. Neben den oben genannten sind auch andere Verstellelemente für die Relativverschiebung geeignet.

In weiteren Ausführungsformen kann die Fixiervorrichtung mindestens eine Befestigungseinrichtung aufweisen, die jeweils dazu eingerichtet ist, eine Verschiebung der inneren bzw. äußeren Schanzschrauben zu verhindern oder zu ermöglichen. Mit der Befestigungseinrichtung können die inneren bzw. äußeren Schanzschrauben am bzw. relativ zum Grundkörper fixiert werden, so dass die Fixiervorrichtung insgesamt bezogen auf den Patienten an Ort und Stelle verbleibt, die der Operateur festgelegt hat. Für den Heilungsprozess ist dies wesentlich, denn nur das Knochenfragment an der/den variabel am Grundkörper angebrachten Schanzschraube(n) wird in kleinen Schritten nach der ersten Anbringung weg vom Knochen bewegt, so dass neues Knochengewebe nach den Prinzipien der Kallusdistraktion generiert werden kann. Wichtig ist hierbei, dass sowohl die äußeren als auch die innere(n) Schanzschraube(n) die Festlegungsfunktion übernehmen können. In besonderen Ausführungsformen können auch alle Schanzschrauben eine solche Befestigungseinrichtung aufweisen, beispielsweise um eine Selbstverstellung der Verschiebeeinrichtung oder eine unerwünschte Verstellung durch den Patienten oder andere Personen zu verhindern.

Dabei kann die Befestigungseinrichtung bevorzugt eine Bohrung auf einer Seite des Grundkörpers und ein darin aufgenommenes Befestigungsmittel aufweisen. Ein Gewindestift oder dergleichen kann dann beispielsweise in die mit einem entsprechenden Gewinde versehene seitliche Bohrung im Grundkörper eingeführt und per Innensechskant oder eine ähnliche Verschraubungsart derart mit der Schanzschraube kraft- oder formschlüssig ineinandergreifen, dass diese im Wesentlichen senkrecht zu ihrer Bewegungsrichtung geklemmt und so an ihrer Bewegung gehindert wird. Es ist anzumerken, dass auch andere Klemmmechanismen die lösbare Festlegung der Schanzschrauben bewirken können, beispielsweise ein Hebelmechanismus, ein Spannkonus oder ein Exzentermechanismus.

Die seitliche Bohrung für die Befestigungseinrichtung ist nicht zu verwechseln mit den Bohrungen von der Oberseite auf die Unterseite des Grundkörpers zur Aufnahme der Schanzschrauben. Diese als vertikal bezeichneten Bohrungen müssen nicht durchgängig sein. Die Durchgängigkeit weist allerdings den Vorteil auf, dass der Grundkörper mit den durchgängigen vertikalen Bohrungen als Bohrlehre verwendet werden kann. Auf diese Weise können die Ansatzpunkte für die Schanzschrauben am Knochen exakt markiert werden.

In weiteren vorteilhaften Ausführungsformen können die inneren und/oder äußeren Schanzschrauben an ihrem oberen Ende ein metrisches Gewinde und an ihrem unteren Ende ein zur Knochenbefestigung geeignetes Gewinde aufweisen. Auch andere Gewindearten außer metrischen Gewinden können zum Einsatz kommen. Die Gewinde am oberen Ende der Schanzschrauben bieten eine relativ einfache Verschiebemöglichkeit durch die Verschiebeeinrichtung mittels der oben genannten Verstellelemente wie Rändelschrauben usw. Auch eine exakte Distanzeinstellung wird damit möglich. Die Gewinde am unteren Ende der Schanzschrauben sind dafür eingerichtet, einfach das äußere Knochengewebe zu durchdringen und in das Knocheninnere einzugreifen. Dafür weisen die Schanzschrauben im Allgemeinen eine scharfe bzw. abgeschrägte Spitze auf, die das Eindringen in den Knochen noch weiter erleichtert. Gegenüber Stiften ohne Gewinde bieten die mit Knochengewinde ausgestatteten Schanzschrauben eine wesentlich bessere Anhaftung und Fixierung am Knochen.

Es ist anzumerken, dass die Schanzschrauben im oberen Bereich nicht notwendigerweise einen runden Querschnitt aufweisen müssen. So kann der Querschnitt z. B. polygonförmig sein. Beispielsweise können sie auch in Längsrichtung einseitig oder mehrseitig abgeflacht sein und so mit der Verschiebeeinrichtung zusammenwirken, so dass sich eine relativ zum Grundkörper verdrehsichere Verschiebung ergibt. Ein Beispiel für einen nicht runden Querschnitt im oberen Bereich ist eine Abflachung auf zwei gegenüber liegenden Seiten, wobei das (metrische) Gewinde auf den verbleibenden Außenflächen weiter vorhanden ist. Bei Ineinandergreifen einer am Grundkörper angebrachten Stellmutter mit der Schanzschraube und bei anschließendem Drehen der Stellmutter bewegt sich der Grundkörper entlang der Schanzschraube vertikal nach oben oder unten, wobei die Schanzschraube in ihrer Längsrichtung konstant zum Grundkörper ausgerichtet bleibt, d. h. es findet keine Drehung der Schanzschraube relativ zum Grundkörper statt. Diese Wirkung kann auch mit anderen geometrischen Strukturen der Schanzschraube mit den zugehörigen Verstellelementen der Verschiebeeinrichtung erzielt werden. Hierbei ist es auch denkbar, dass die Schanzschrauben im oberen, geführten Bereich kein Gewinde, sondern nur eine Führungsgeometrie aufweisen, d. h. Führung und Gewinde sind in Längsrichtung getrennt.

In bevorzugten Ausführungsformen kann die Verschiebeeinrichtung ein Stellelement aufweisen, das in einer Ausnehmung des Grundkörpers aufgenommen ist, wobei eine Bewegung des Stellelements eine Verschiebung der inneren bzw. äußeren Schanzschraube relativ zum Grundkörper bewirkt. Für die Anwendung der Fixiereinrichtung ist es wichtig, dass die Distanz des extrahierten Knochenfragments zum festgelegten Knochen möglichst exakt einstellbar ist, denn nur so kann die Behandlung optimiert werden. Stellelemente wie Rändelschrauben mit einer Skala oder ähnliche Einheiten mit geeigneten Maßstäben sind dafür besonders gut eingerichtet. Dabei ist auch zu berücksichtigen, dass sich die einmal vorgenommene Einstellung nicht selbständig verändern lassen sollte, um keine Rückstellung oder Rückwärtsbewegung entgegen der Distraktion zuzulassen. Die Verschiebeeinrichtung kann dafür eine entsprechende Reibung beispielsweise in den Schraub- oder Klemmelementen vorsehen, so dass sich einmal eingestellte Distanzwerte nicht ohne Weiteres von allein verändern können. Alternativ kann die Verschiebeeinrichtung auch über eine zusätzliche Klemmung verfügen.

In einer weiteren Ausführungsform kann der Grundkörper seitlich mindestens eine Ausnehmung aufweisen. Dadurch ist eine Materialeinsparung möglich, die die gesamte Fixiervorrichtung leichter macht und die Handhabung verbessern kann. Vorstellbar ist beispielweise, dass die Ausnehmungen derart ausgebildet sind, dass sich der Grundkörper an die Kontur einer Hand anschmiegt. In ähnlicher Weise kann die Oberfläche des Grundkörpers an den Kanten, auch knochenzugewandt, abgerundet oder angefast sein, um weitere scharfe Kanten abzumildern.

In besonders bevorzugten Ausführungsformen kann die Verschiebeeinrichtung einen länglichen Innenkörper umfassen, wobei der Innenkörper vorzugsweise in einem Aufnahmeraum auf der Unterseite des Grundkörpers aufnehmbar ist. Dabei kann die mindestens eine innere Schanzschraube an dem Innenkörper angebracht sein. In einer solchen Konfiguration mit mindestens einer inneren Schanzschraube am Innenkörper ist die oben beschriebene Alternative b) verwirklicht, d. h. durch die Betätigung der Verschiebeeinrichtung am Grundkörper, an dem die äußeren Schanzschrauben festgelegt sind, wird der Innenkörper relativ zum Grundkörper bewegt. Mit anderen Worten, die Bewegung des Innenkörpers relativ zum Grundkörper bewirkt eine gleichzeitige und gleichmäßige Verschiebung der am Innenkörper festgelegten inneren Schanzschrauben. Bei Betätigung der Verschiebeeinrichtung bewegt sich hier nicht der gesamte Grundkörper zusammen mit der mindestens einen inneren Schanzschraube wie bei der oben beschriebenen Alternative a), sondern nur der Innenkörper.

Dabei kann die Verschiebeeinrichtung eine Gewindeschraube umfassen, die den Grundkörper mit dem Innenkörper verbindet und einen Rändelkopf aufweist, und dazu eingerichtet ist, durch Drehen den Innenkörper zusammen mit dem Knochensegment relativ zum Grundkörper vom Gesamtknochen weg oder wieder zum Knochen hin zu bewegen. Wie bereits oben beschrieben kann dadurch eine gleichmäßige Verschiebung mit einer möglichst exakten Maßeinstellung erzielt werden. Es sind jedoch auch andere Verstellmechanismen möglich, die nicht auf einem Gewinde bzw. einer Drehung beruhen wie beispielweise eine oben glatte Schanzschraube, die im Bereich des Grundkörpers verschieblich geklemmt wird. Ein Einrast- oder Ratschenmechanismus kann dafür verwendet werden.

In ausgewählten Ausführungsformen kann der Grundkörper zumindest teilweise als rohrförmiger Hohlkörper ausgebildet sein. Dabei kann der Querschnitt kreisförmig, ellipsenförmig, quadratisch, rechteckig oder polygonal ausgebildet sein. Die Verschiebeeinrichtung und die weiteren Elemente können dann durch entsprechende fest eingebrachte Einsätze ausgebildet sein, um die Funktion des Elements zu gewährleisten. Derartige Einsätze können dann beispielsweise geklebt oder auf andere Weise am Grundkörper befestigt sein.

Alternativ kann der Grundkörper auch aus vollem Material ausgebildet sein wie z. B. einem Metall oder einer Metalllegierung. Auch können Kunststoffe als Vollmaterial zum Einsatz kommen, die eine entsprechende Eignung für medizinische Produkte aufweisen, beispielsweise Duroplaste oder Thermoplaste sowie faserverstärkte Kunststoffe wie GFK oder CFK.

In bestimmten vorteilhaften Ausführungsformen können zumindest der Grundkörper und/oder der Innenkörper oder auch andere Bestandteile mittels 3D-Druck hergestellt sein. 3D-Druck ist heutzutage ein schnelles und auch günstiges Herstellungsverfahren. Es zeichnet sich dadurch aus, dass unterschiedliche Materialien am gleichen Werkstück zum Einsatz kommen und Vorrichtungen wie die erfindungsgemäße Fixiervorrichtung in einem Vorgang hergestellt werden können. Mit 3D-Druck sind vor allem auch geeignete, stabile Strukturen wie Skelettstrukturen, Wabenstrukturen und andere möglich, die in herkömmlichen Herstellungsverfahren vergleichsweise aufwändig und teuer sind.

In weiteren bevorzugten Ausführungsformen kann die Verschiebeeinrichtung mittels Aktuator betreibbar sein. Damit ist eine exakte, gegebenenfalls automatische Einstellung der Verschiebeeinrichtung möglich. Der Aktuator kann zusätzlich auch manuell einstellbar sein. Elektrische Antriebe für den Aktuator sind bevorzugt, es können auch andere Arten von Aktuatoren zum Einsatz kommen.

Die vorliegende Erfindung wird nachfolgend anhand von bevorzugten Beispielen und Ausführungsformen unter Bezugnahme auf die beigefügten Figuren beschrieben, in denen:
- Fig. 1: eine perspektivische Ansicht einer ersten Ausführungsform der erfindungsgemäßen Fixiervorrichtung ist;
- Fig. 2: eine Querschnittansicht und Seitenansicht eines Ausschnitts der Fixiervorrichtung aus Fig. 1 darstellt;
- Fig. 3: eine perspektivische Detailansicht einer zweiten Ausführungsform der erfindungsgemäßen Fixiervorrichtung ist;
- Fig. 4: eine Seitenansicht einer Schanzschraube gemäß einer bevorzugten Ausführungsform der erfindungsgemäßen Fixiervorrichtung darstellt;
- Fig. 5: eine perspektivische Ansicht einer dritten Ausführungsform der erfindungsgemäßen Fixiervorrichtung ist;
- Fig. 6: eine schematische Detailansicht einer Verschiebeeinrichtung gemäß der Ausführungsform aus Fig. 5 zeigt;
- Fig. 7: eine Seitenansicht einer Schanzschraube mit einem Teil einer Verschiebeeinrichtung gemäß einer weiteren Ausführungsform zeigt;
- Fig. 8: eine Teilseitenansicht einer Schanzschraube mit einem Teil eine Verschiebeeinrichtung gemäß einer weiteren Ausführungsform ist; und
- Fig. 9: eine schematische perspektivische Ansicht eines vierten Ausführungsform der erfindungsgemäßen Fixiervorrichtung darstellt.

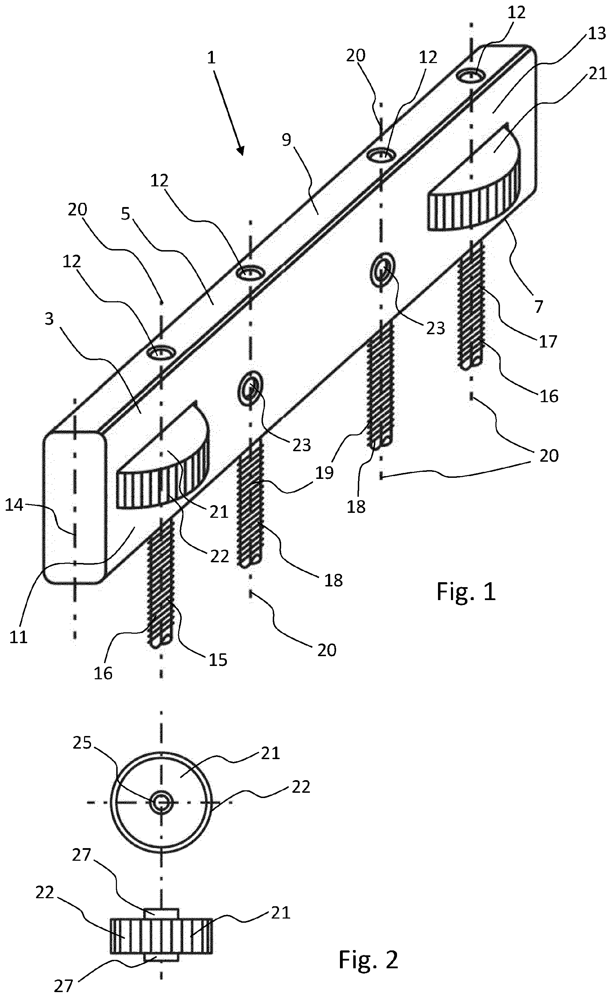

Fig. 1 zeigt eine schematische perspektivische Ansicht einer ersten Ausführungsform der erfindungsgemäßen Fixiervorrichtung. Die Fixiervorrichtung 1 umfasst einen in dieser Ausführungsform im Wesentlichen quaderförmigen Grundkörper 3, der eine Oberseite 5, eine Unterseite 7, einen Mittelabschnitt 9, ein erstes Ende 11 sowie ein zweites Ende 13 aufweist. An dem ersten Ende 11 ist in einer hier durchgängigen Bohrung 12 eine erste äußere Schanzschraube 15 angebracht und zwar derart, dass sie von der Unterseite 7 des Grundkörpers 3 herausragt. In gleicher Art und Weise ist an dem zweiten Ende 13 an einer ebenfalls durchgängigen Bohrung 12 auf der Unterseite 7 des Grundkörpers 3 eine zweite äußere Schanzschraube 17 angeordnet. Die beiden äußeren Schanzschrauben 15, 17 sind im Wesentlichen parallel zueinander angeordnet mit Drehachsen 20, die in der hier dargestellten Ausführungsform in der Symmetrieebene 14 liegen, die sich von der Oberseite 5 zur Unterseite 7 des Grundkörpers 3 erstreckt.

In der in Fig. 1 dargestellten Ausführungsform ist die Oberseite 5 des Grundkörpers 3 bei der transversalen Distraktion dem Knochen abgewandt und die Unterseite 7 des Grundkörpers 3 entsprechend dem Knochen mit den Schanzschrauben zugewandt. Der Mittelabschnitt 9 weist auf der Unterseite 7 zwei innere Schanzschrauben 19 auf, die ebenfalls in Bohrungen 12 mit der Drehachse 20 angeordnet sind. Auch die Bohrungen 12 der inneren Schanzschrauben 19 sind parallel zueinander und in der dargestellten Ausführungsform auch parallel zu den Bohrungen 12 der äußeren Schanzschrauben 15, 17. Diese Parallelität ist jedoch nicht zwingend. Sowohl die äußeren als auch die inneren Schanzschrauben sind in ihrem oberen Bereich, d. h. dem Bereich, der mit dem Grundkörper 3 in Eingriff steht, zumindest teilweise mit einem metrischen (Außen-) Gewinde 18 versehen. Mit anderen Worten, die nicht mit der Verschiebeeinrichtung in Eingriff stehenden Schanzschrauben können im oberen Bereich auch glatt ausgebildet sein.

Die erste äußere Schanzschraube 15 sowie die zweite äußere Schanzschraube 17 stehen in der hier dargestellten Ausführungsform wirksam mit jeweils einer Verschiebeeinrichtung 21 in Verbindung, die dazu eingerichtet ist, den Grundkörper 3 und damit die fest mit dem Grundkörper 3 verbundenen inneren Schanzschrauben 19 in Vertikalrichtung relativ zu den äußeren Schanzschrauben 15, 17 zu verschieben. Dazu umfasst die Verschiebeeinrichtung 21 jeweils ein Stellrad, das in einer Ausnehmung des Grundkörpers 3 aufgenommen ist. Die Ausnehmung erstreckt sich in der hier dargestellten Ausführungsform von einer Seite zur anderen Seite des Grundkörpers 3. Es ist anzumerken, dass dies nicht notwendigerweise in dieser Art und Weise symmetrisch sein muss, sondern auch beispielsweise einseitig ausgeführt sein kann, so dass die Verschiebeeinrichtung bzw. das Stellrad 21 nur auf einer Seite des Grundkörpers 3 vorspringen. Eine Drehung des Stellrads 21 bewirkt eine axiale Verschiebung des Grundkörpers 3 mitsamt den inneren Schanzschrauben 19, die ja am zu distrahierenden Knochenfragment befestigt sind, relativ zu den äußeren Schanzschrauben 15, 17, die bei der Verschiebung ortsfest bleiben. Dies wird dadurch erreicht, dass das Stellrad 21 in der Mitte eine Gewindebohrung 25 aufweist, die in das Außengewinde 16 auf dem oberen Abschnitt der äußeren Schanzschrauben 15, 17 eingreift. Auf dem Außenumfang weist das Stellrad 21 eine Rändelung 22 oder eine andere Art von aufgerauter Grifffläche auf, die dazu eingerichtet ist, beispielsweise mit den Fingern eine Bedienperson berührt zu werden, so dass sich das Stellrad 21 um die Drehachse 20 der Schanzschraube 15, 17 dreht. Bei Bewegung des Stellrades drehen sich folglich weder die inneren noch die äußeren Schanzschrauben um ihre Drehachse 20.

Damit das Stellrad 21 im Inneren des Grundkörpers 3 um die Drehachse 20 drehbar gelagert ist, weist es auf seiner Ober- und/oder Unterseite vorzugsweise ein Führungselement 27 auf, wie unter Bezugnahme auf Fig. 2 näher beschrieben. Die beiden inneren Schanzschrauben 19 sind in ihrem oberen Bereich fest mit dem Grundkörper 3 verbunden, d. h. beispielsweise in den Bohrungen 12 per Gewinde verschraubt oder geklemmt. Sobald die vertikale Position der inneren Schanzschrauben 19 festgelegt ist, werden die inneren Schanzschrauben 19 bevorzugt durch eine Befestigungseinrichtung fixiert. In der hier dargestellten Ausführungsform ist die Befestigungseinrichtung durch eine Bohrung 23 ausgebildet, die im Wesentlichen senkrecht zu den Drehachsen 20 steht und ein Innengewinde aufweist, in das eine entsprechende Schraube oder ein Gewindestift eingeführt werden kann und bei Anschlag auf die innere Schanzschraube 19 zu deren axialer Festlegung führt. Die Befestigungseinrichtung 23 kann jedoch während oder nach einem bestimmungsgemäßen Einsatz der Fixiervorrichtung 1 wieder gelöst werden. Hinsichtlich der Befestigungseinrichtung 23 ist es denkbar, dass diese nicht durch eine Bohrung mit eingesetzter Schraube, sondern durch eine andere Art der Klemmung an ihrer axialen Bewegung gehindert wird. Beispiele für derartige Klemmung sind Keile, Spann- oder Klemmkonus, Klemmpfropfen, Klemmungen mit Bajonett-Verschluss ähnlich einer Gartenschlauchklemmung und dergleichen.

Fig. 2 zeigt eine Querschnittansicht eine Seitenansicht eines Ausschnitts der Verschiebeeinrichtung aus Fig. 1. Man erkennt in der Draufsicht das Stellrad 21 mit seiner Gewindebohrung 25 in der Drehachse 20 und auf seinem äußeren Umfang eine Rändelung 22, die dazu eingerichtet ist, dass ein Benutzer das Stellrad 21 mit seinen Fingern verdrehen kann. In der Seitenansicht der Fig. 2 ist ebenfalls die Rändelung bzw. der Griffbereich 22 in Umfangsrichtung erkennbar. Auf der Oberseite bzw. der Unterseite des Stellrad 21 sind hier Führungselemente 27 angeordnet, die dazu eingerichtet sind, dass sich das Stellrad 21 innerhalb der Ausnehmung des Grundkörpers 3 im Wesentlichen spielfrei drehen kann. Es ist ebenfalls möglich, dass hier eine ordentliche Lagerung mittels eines Kugel- oder Nadellagers vorgesehen ist. Aus Gründen der Einfachheit der Konstruktion wird bei der hier dargestellten Ausführungsform jedoch auf komplexe Lagerungen des Stellrads 21 verzichtet. Es ist anzumerken, dass das Stellrad 21 auch ohne Führungselement(e) 27 im Grundkörper 3 geführt sein kann.

Für die Funktion der erfindungsgemäßen Fixiervorrichtung in der hier dargestellten Ausführungsform ist wesentlich, dass die äußeren Schanzschrauben 15, 17 fest in den Bohrungen 12 aufgenommen sind und sich bei Bewegung der Verschiebeeinrichtung 21, also hier bei Drehung des Stellrads, die Verschiebeeinrichtung 21 mit dem Grundkörper 3 und den daran befestigten inneren Schanzschrauben 19 in der Höhe relativ zu den sich nicht drehenden äußeren Schanzschrauben 15, 17 bewegt. Eine weitere Möglichkeit, die Eigenschaft der drehfesten axialen Lagerung der äußeren Schanzschrauben 15, 17 in dieser Ausführungsform konstruktiv herzustellen, ist das Abflachen der äußeren Schanzschrauben in dem Bereich, in dem sie im Inneren des Grundkörpers 3 aufgenommen bzw. geführt sind, d.h. in den Bohrungen 12. Entsprechend können die Bohrungen 12 dann einen zum Querschnitt der Schanzschrauben komplementären Innenquerschnitt aufweisen. Es versteht sich, dass außer dem Abflachen auch andere geometrische Ausgestaltungen der äußeren Schanzschrauben möglich sind. Ebenfalls sind andere Formen und Gegenformen der Verschiebeeinrichtung 21 bzw. der oberen Enden der äußeren Schanzschrauben 15, 17 möglich. Der Grundkörper 3 kann damit bezüglich der äußeren Schanzschrauben 15, 17 derart drehfest festgelegt sein, dass ein axiales Verdrehen der Schanzschrauben 15, 17, nicht möglich ist, sondern nur ein vertikales Verschieben in Längsrichtung der Schanzschrauben, bewirkt durch Drehen der Verschiebeeinrichtung 21.

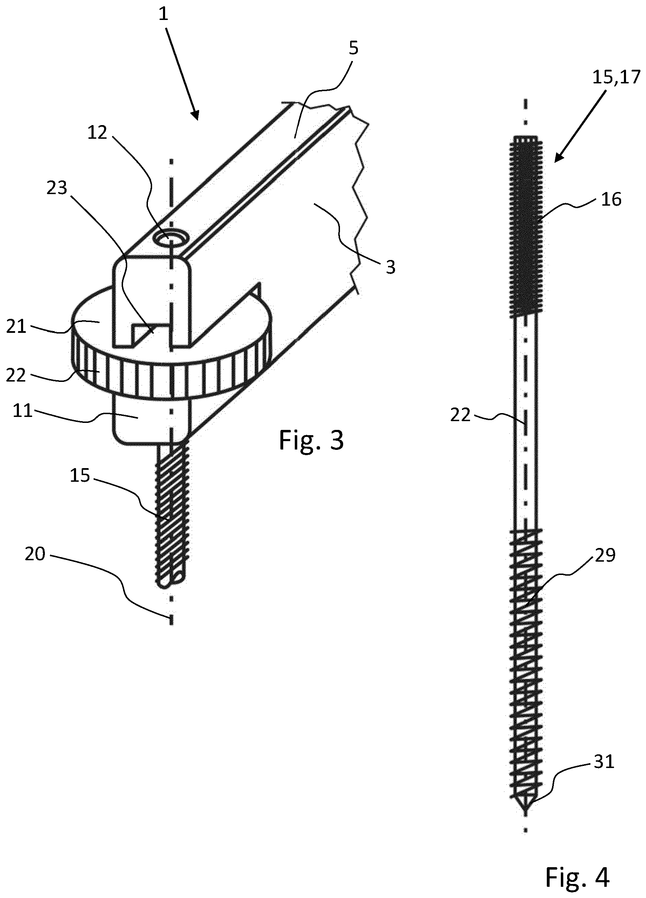

Fig. 3 zeigt eine perspektivische Detailansicht einer zweiten Ausführungsform der erfindungsgemäßen Fixiervorrichtung. Der Unterschied zur ersten Ausführungsform aus Fig. 1 liegt darin, dass die äußeren Schanzschrauben 15, 17 ganz außen an den ersten bzw. zweiten Enden 11, 13 angeordnet sind. Die Ausgestaltung und Befestigung der inneren Schanzschrauben 19 ist im Wesentlichen identisch, weshalb diese in Fig. 3 nicht abgebildet sind. Man erkennt in Fig. 3 das erste Ende 11, an dem in einem geringen Abstand zur Stirnseite des Grundkörpers 3 die Bohrung 12 mit der darin eingesetzten äußeren Schanzschraube 15 vorgesehen ist. Das Stellrad 21 aus der ersten Ausführungsform weist im Inneren denselben Aufbau auf. Anders als bei der ersten Ausführungsform ragt jedoch das Stellrad 21 über die Stirnseite des Grundkörpers 3 hinaus. Im Grundkörper 3 ist die Ausnehmung zur Aufnahme des Stellrads 21 derart vergrößert, dass das Stellrad 21 mit dem (in Fig. 2 dargestellten) Führungselement 27 von der Stirnseite des Grundkörpers 3 eingeführt werden kann. Diese Ausnehmung kann auch dazu verwendet werden, eine Befestigungseinrichtung 23 aufzunehmen, um ein axiales Verdrehen des Stellrads 21 zu verhindern. Beispielsweise kann als Befestigungseinrichtung 23 ebenfalls ein stirnseitig oder von der Seite eingebrachter Gewindestift bzw. ein anderes Klemmelement verwendet werden, der sich zwischen Grundkörper 3 und der Oberfläche des Stellrads 21 klemmt. Andere Ausgestaltungen der Klemmeinrichtung sind ebenfalls möglich. Ein Vorteil der zweiten in Fig. 3 dargestellten Ausführungsform ist, dass der Grundkörper kürzer ausgeführt werden kann und die Angriffsfläche bzw. die Umfangsfläche des Stellrads des 21 bezogen auf die erste Ausführungsform vergrößert ist. Damit wird die Handhabung etwas verbessert. Darüber hinaus kann beispielsweise auch eine auf der Umfangsfläche des Stellrads 21 angebrachte Messskala besser lesbar sein, die dazu dient die axiale Verschiebung der äußeren Schanzschrauben 15, 17 möglichst exakt einzustellen.

Fig. 4 zeigt eine Seitenansicht einer inneren oder äußeren Schanzschraube gemäß einer bevorzugten Ausführungsform erfindungsgemäßen Fixiervorrichtung. Eine hier verwendete Schanzschraube ist üblicherweise aus einem Medizin-kompatiblen Kunststoff oder Metall bzw. einer entsprechend geeigneten Metalllegierung ausgebildet und weist im oberen Abschnitt ein Gewinde 16, einen im wesentlichen glatten Mittelabschnitt sowie im unteren Abschnitt ein sogenanntes Knochengewinde 29 auf. Wie bereits oben erwähnt, kann das Material auch ein Kunststoff sein, der die Anforderungen an die medizinische Kompatibilität und die Verwendbarkeit bei einer transversalen Knochendistraktion erfüllt. Es versteht sich, dass nur die Schanzschrauben im oberen Bereich ein Gewinde aufweisen müssen, wenn sie in diesem Bereich mit einer Verschiebeeinrichtung mit Innengewinde zur Bewegung in Axialrichtung in Eingriff stehen. Daher können die Schanzschrauben im oberen Bereich auch ohne Gewinde ausgeführt sein. Das Knochengewinde 29 weist eine relativ hohe Gewindesteigung auf, die dazu dient, die Schanzschraube mit möglichst wenigen Umdrehungen in den Knochen zu treiben. Für die Zwecke des präzisen Ansetzens und möglichst einfachen Einbohrens in die Knochenoberfläche weist die Schanzschraube bevorzugt an ihrem unteren Ende eine abgeschrägte, scharfe Spitze auf. Das Gewinde 16 im oberen Bereich der Schraube ist in den hier vorgestellten Ausführungsformen als metrisches Gewinde ausgebildet, beispielsweise mit einem Außendurchmesser von 4 mm. Es ist ebenfalls möglich, statt eines metrischen Gewindes eine andere Art von Gewinde zu verwenden, beispielsweise ein Zoll-Gewinde. Die Längen der entsprechenden Gewindeabschnitte sind auf die Art des zu distrahierenden Knochens abgestimmt und damit auf die Länge der Knochendistraktion. Bei einer Distraktion der Tibia beträgt das Distraktionsmaß typischerweise zwischen 5 mm und 25 mm, bevorzugt zwischen 10 mm und 20 mm. Wie bereits oben erwähnt muss der Querschnitt der Schanzschraube im oberen Bereich nicht notwendigerweise rund sein, sondern kann wegen der Notwendigkeit der Drehfestigkeit im Querschnitt unregelmäßig bzw. unsymmetrisch sein. Beispielsweise kann die Schanzschraube in Längsrichtung auf zwei gegenüberliegenden Seiten abgeflacht sein oder ein D-Profil aufweisen, d. h. auf einer Seite glatt und im verbleibenden Umfang mit Gewinde.

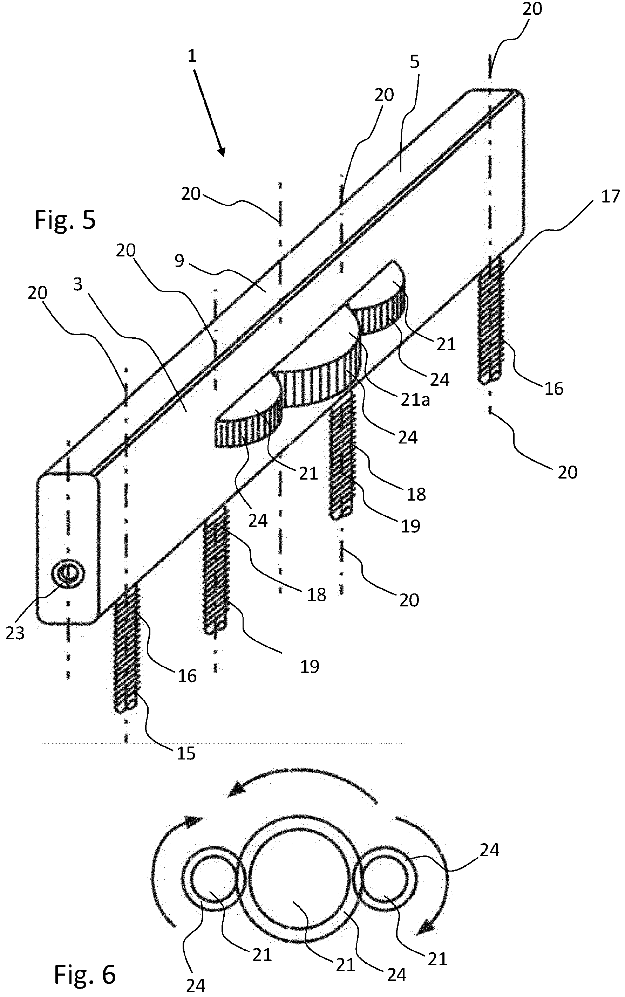

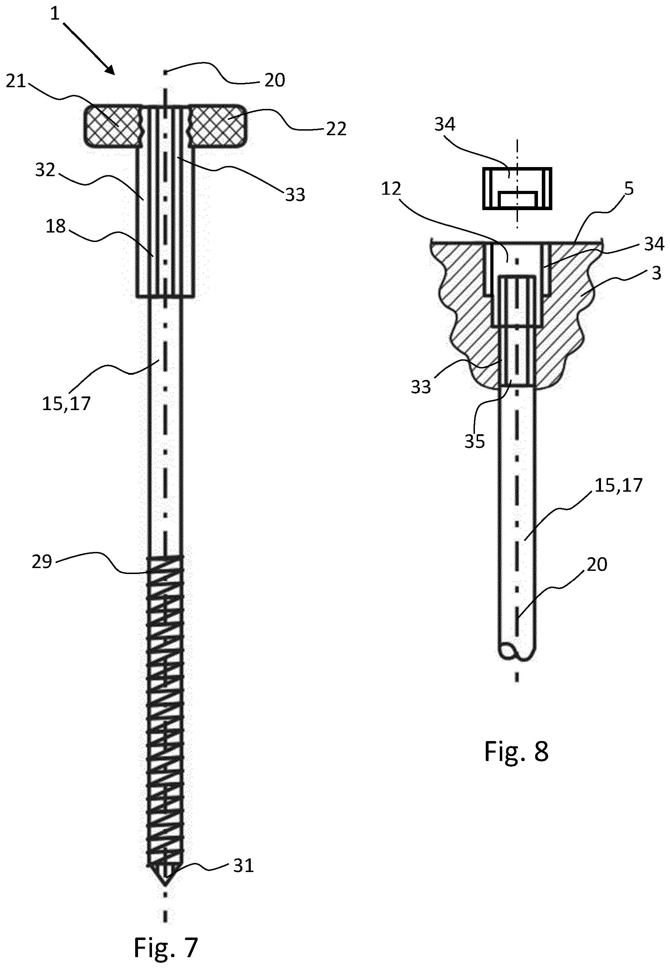

Fig. 5 zeigt eine schematische perspektivische Ansicht einer dritten Ausführungsform der erfindungsgemäßen Fixiervorrichtung. Der Grundaufbau der Fixiervorrichtung 1 in dieser Ausführungsform ist ähnlich zu dem der ersten Ausführungsform aus Fig. 1, insbesondere weist der Grundkörper 3 im Wesentlichen die gleiche Form auf. Bei der Beschreibung der in Fig. 5 dargestellten dritten Ausführungsform wird daher besonders auf die Unterschiede zu der ersten Ausführungsform aus Fig. 1 eingegangen. Die erste äußere Schanzschraube 15 ist ebenfalls an dem ersten Ende 11 des Grundkörpers 3, und die zweite äußere Schanzschraube 17 ist ebenfalls an dem zweiten Ende 13 des Grundkörpers 3 angebracht, und zwar auch von der Unterseite 7 jeweils in Bohrungen 12, die hier wegen der perspektivischen Darstellung nicht sichtbar sind, jedoch auch durchgängig ausgeführt sein können. Im Unterschied zur ersten Ausführungsform können die Bohrungen 12 auch nicht durchgängig ausgeführt sein, d. h. sie treten nicht durch die Oberseite 5 des Grundkörpers 3 hindurch und bilden ein Sackloch, so dass die Oberseite 5 eine Ebene glatte Oberfläche ohne Ausnehmungen oder Vorsprünge bildet. Die erste äußere Schanzschraube 15 und die zweite äußere Schanzschraube 17 sind in den Bohrungen 12 aufgenommen und werden jeweils von einer Befestigungseinrichtung 23 fixiert bzw. an Ort und Stelle gehalten. Ähnlich zu der Beschreibung der Befestigungseinrichtung 23 bezogen auf die erste Ausführungsform in Fig. 1 umfasst die Befestigungseinrichtung 23 hier eine Gewindebohrung, die von der Stirnseite des Grundkörpers 3 parallel zur Längsachse des Grundkörpers 3 bis in die Bohrung 12 reicht. In diese Gewindebohrung eingeführt ist beispielsweise ein Gewindestift mit einem Innensechskant-Ansatz, so dass er von einem entsprechenden Werkzeug hinein- oder wieder herausgeschraubt werden oder von oben geklemmt werden kann (siehe Fig. 8). Auch die zweite äußere Schanzschraube 17 wird von einer (hier nicht sichtbaren) Befestigungseinrichtung 23 in gleicher Art fixiert. Hier ist es auch möglich, dass die zur Befestigungseinrichtung 23 gehörende Bohrung nicht auf der Stirnseite, sondern auf der seitlichen Fläche des Grundkörpers 3 angeordnet ist.

Entsprechend sind bei dieser dritten Ausführungsform in Fig. 5 die inneren Schanzschrauben 19 dazu eingerichtet, dass sie durch die Verschiebeeinrichtung 21 relativ zum Grundkörper 3 in ihrer Längsrichtung entlang der Achsen 20 verschoben werden können, und zwar mittels eines zentralen Stellrads 21a, das zwischen zwei im Wesentlichen identischen Stellrädern angeordnet ist und damit zusammenwirkt. Die Verschiebeeinrichtung 21 umfasst in dieser dritten Ausführungsform jeweils ein Stellrad, das wie in der ersten Ausführungsform koaxial mit der entsprechenden Schanzschraube ausgerichtet und im Wesentlichen mittig bezogen auf die Symmetrieachse 14 innerhalb des Grundkörpers 3 angeordnet ist. Wie unter Bezugnahme auf Fig. 1 beschrieben, bewirkt eine Drehung des Stellrads eine Längsverschiebung der inneren Schanzschraube 19 entlang der Achse 20, wobei die Schanzschraube 19 sich während der Verschiebung um die Achse 20 nicht dreht, sondern drehfest angeordnet bleibt. Die Anordnung der zweiten inneren Schanzschraube 19 ist identisch zur ersten Schanzschraube 19 einschließlich des Stellrades. Die Drehung der beiden Stellräder und damit die vertikale Verschiebung der beiden inneren Schanzschrauben 19 wird durch die Drehung des Stellrades 21a bewirkt, das auf seiner Umfangsfläche eine Verzahnung 24 aufweist, die mit den Verzahnungen 24 auf der Umfangsfläche der beiden angrenzenden Stellräder ineinandergreift. Das hier etwas größer ausgebildete Stellrad 21a ist innerhalb des Grundkörpers 3 ebenfalls mittig bezogen auf die Symmetrieebene 14 angeordnet und kann auch (hier nicht sichtbare) Führungselemente 27 umfassen, die dazu dienen, dass sich das Stellrad 21a innerhalb des Grundkörpers 3 in ausreichender Weise bewegen und drehen kann. Beide äußeren Stellräder greifen jeweils mit ihre Verzahnung 24 in jene des Stellrades 21a ein und bewirken durch die exakt gleich große Geometrie eine synchrone vertikale Verschiebung der beiden inneren Schanzschrauben 19 bezogen auf den Grundkörper 3. Insbesondere das zentrale Stellrad 21a kann auf seiner Oberseite oder auch auf seinem Umfang einen Maßstab bzw. eine Skala aufweisen, auf der der Wert der vertikalen Verschiebung der beiden inneren Schanzschrauben 19 abgelesen werden kann. Damit kann medizinisches Personal oder auch der Patient selbst den Wert der transversalen Distraktion ablesen bzw. einstellen. Es versteht sich, dass auch andere einfache Verstellmechanismen von der Verschiebeeinrichtung 21 umfasst sein können, die eine gleiche Wirkung, nämlich die synchrone vertikale Verschiebung der inneren Schanzschrauben 19 erzielen.

Bei einer weiteren, zur Ausführungsform der Fig. 5 ähnlichen Ausführungsform kann das zentrale Stellrad 21a auch in einer anderen Ebene wie die mit den inneren Schanzschrauben 19 axial ausgerichteten Stellräder angeordnet sein. Dazu weist das Stellrad 21a im Inneren des Grundkörpers 3 beispielsweise einen verzahnten Schaft auf, der in die Verzahnung der beiden Stellräder eingreift. Auf diese Weise kann eine Übersetzung erzielt werden, womit die Verschiebeeinrichtung leichtgängiger und exakter einstellbar wird.

In Fig. 6 ist eine schematische Detailansicht von Bestandteilen der Verschiebeeinrichtung 21 gemäß der in Fig. 5 dargestellten dritten Ausführungsform gezeigt. Eine Drehung des Stellrades 21a gegen den Uhrzeigersinn bewirkt eine identische, gegensinnige Drehung der äußeren Stirn- bzw. Stellräder, deren Verzahnung 24 in die umfängliche Verzahnung 24 des Stellrades 21a greift. Eine Drehung im Uhrzeigersinn des Stellrads 21a bewirkt analog eine Drehung der Stirnräder gegen den Uhrzeigersinn.

Fig. 7 ist eine Seitenansicht einer Schanzschraube mit einem Teil einer Verschiebeeinrichtung gemäß einer weiteren Ausführungsform der erfindungsgemäßen Fixiervorrichtung. Wie in den zuvor beschriebenen Ausführungsformen geschildert, können die axial verschiebbaren Schanzschrauben entweder innen am Mittelabschnitt 9 oder an den äußeren Enden 11, 13 des Grundkörpers angeordnet sein. Die in Fig. 7 abgebildete Schanzschraube 15, 17 mit Verschiebeeinrichtung 21 kann beispielsweise jeweils als äußere Schanzschraube bei der ersten Ausführungsform eingesetzt werden, die in Fig. 1 dargestellt ist.

Im Unterschied zur ersten Ausführungsform weist das Stellrad der Verschiebeeinrichtung 21 mittig einen Schaft 32 auf, der sich von der knochenzugewandten Unterseite des Stellrads erstreckt und in die entsprechend größere Bohrung 12 im Grundkörper 3 derart aufgenommen ist, dass eine leichtgängige Drehung sowie entsprechende Führung möglich ist. Wie bei den zuvor beschriebenen Ausführungsformen weist die Umfangsfläche des Stellrads 21 eine Rändelung auf, so dass eine Bedienperson die Verschiebeeinrichtung 21 bewegen bzw. drehen kann und dadurch eine Verschiebung des Grundkörpers 3 relativ zu den Schanzschrauben 15, 17 bewirkt wird. Die Verschiebeeinrichtung 21 ist drehbar durch ein entsprechendes Führungselement derart am Grundkörper 3 angebracht, dass sie sich bezüglich der Drehachse 20 drehen kann, aber in Vertikalrichtung, also entlang der Drehachse 20, nicht verschoben werden kann. So sorgt die Drehung der Verschiebeeinrichtung 21 für eine entsprechende Vertikalverschiebung des Grundkörpers 3 bezüglich der Schanzschraube 15, 17.

Hinsichtlich der flachen Oberseite des Stellrads der Verschiebeeinrichtung 21 ist anzumerken, dass diese bündig mit der Oberfläche bzw. Oberseite 5 des Grundkörpers 3 abschließt, damit die gesamte Oberfläche der Fixiervorrichtung 1 vorsprungsfrei und im Wesentlichen eben bleibt. Bei einer nicht ebenen Oberseite 5 des Grundkörpers 3 ist die Kontur der Oberseite des Stellrads entsprechend angepasst, damit der Übergang zwischen den Bestandteilen im Wesentlichen nahtlos und ohne Kante ist.

Fig. 8 ist eine Teilseitenansicht einer Schanzschraube, eingesetzt in eine Bohrung innerhalb des Grundkörpers, gemäß einer weiteren Ausführungsform der erfindungsgemäßen Fixiervorrichtung. Dieser Ausführungsform zeichnet sich dadurch aus, dass die Bohrung 12, wie sie beispielsweise in der ersten Ausführungsform durchgängig durch den Grundkörper 3 ausgebildet ist, angrenzend an die Oberseite 5 einen aufgeweiteten Bereich aufweist, der dazu eingerichtet ist, ein Schraub- oder Klemmelement 34 aufzunehmen. Dieses Schraub- oder Klemmelement 34 soll dazu dienen, die Schanzschraube 19 innerhalb des Grundkörpers 3 zu fixieren. Es weist beispielsweise eine Deckelform mit einem Außengewinde auf, das mit dem Innengewinde der aufgeweiteten Bohrung 12 in Eingriff gebracht werden kann, und ebenso eine Innenfläche, die mit dem oberen Ende der Schanzschraube 19 ineinandergreift und diese klemmt bzw. fixiert. Diese Innenfläche kann beispielsweise abgeschrägt sein, und die Oberseite des Schraub- oder Klemmelements 34 kann eine Innensechskantaufnahme umfassen, damit das Element zur Klemmung in den oberen Abschnitt der Buchung 12 eingebracht und dort festgelegt werden kann. Es versteht sich, dass hier andere Klemmmechanismen zur Anwendung kommen können, die dazu eingerichtet sind, die Schanzschraube 19 vertikal innerhalb der Bohrung 12 des Grundkörpers 3 festzulegen, ohne dass ein Bestandteil dieser Befestigungseinrichtung über die Oberseite 5 des Grundkörpers 3 hinausragt. Vielmehr ist es vorteilhaft, wenn die Oberseite des Schraub- oder Klemmelements 34 bündig mit der Oberseite 5 des Grundkörpers 3 abschließt.

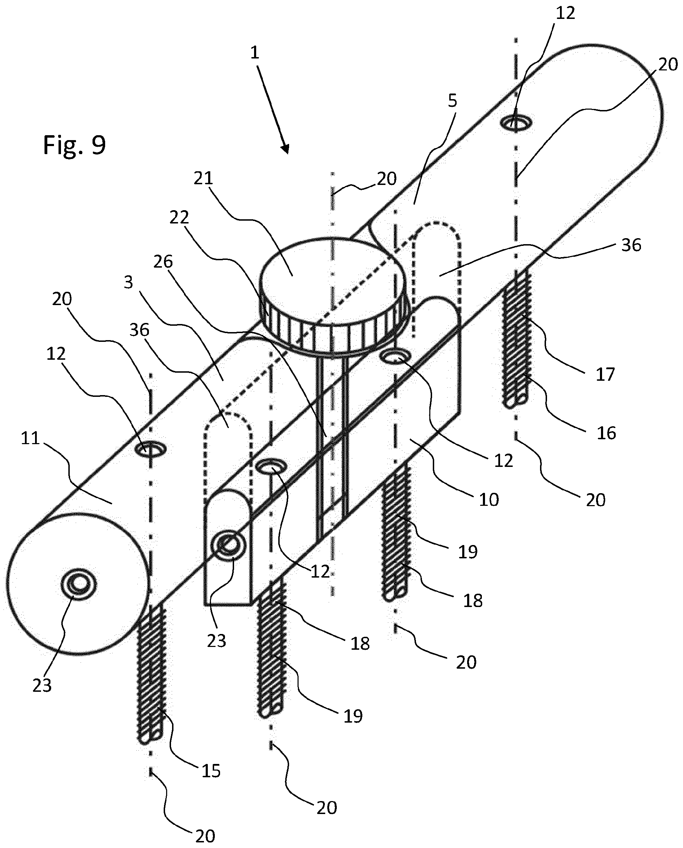

Fig. 9 zeigt eine schematische, perspektivische Ansicht einer bevorzugten vierten Ausführungsform der erfindungsgemäßen Fixiervorrichtung. Im Unterschied zu den zuvor beschriebenen Ausführungsformen umfasst die in Fig. 9 dargestellte Ausführungsform einen zylinderförmigen Grundkörper 3, der entsprechend eine im Wesentlichen runde bzw. abgerundete Oberseite 5 und Unterseite 7 aufweist. Die Anordnung der äußeren bzw. inneren Schanzschrauben ist ähnlich wie bei der dritten Ausführungsform, die in Fig. 5 gezeigt ist. D. h., die äußeren Schanzschrauben 15, 17 sind in den jeweiligen Bohrungen 12 vertikal festgelegt, hier mittels der Befestigungseinrichtung 23, die auf der Stirnseite des Grundkörpers 3 angeordnet ist und hier eine Gewindebohrung darstellt, in die eine Klemmschraube zur Klemmung der Schanzschraube 15 eingebracht ist. Wie bereits beschrieben kann die Befestigungseinrichtung 23 auch seitlich am Grundkörper 3 und entsprechend im Inneren angeordnet sein.

Die beiden inneren Schanzschrauben 19 sind hier jedoch nicht direkt am Grundkörper 3 angebracht, sondern an einem Innenkörper 10, der eine längliche, Quader-ähnliche Form aufweist und auf der Unterseite 7 des Grundkörpers 3 in einem Aufnahmeraum 36 des Grundkörpers 3 aufgenommen ist. Der Aufnahmeraum 36 ist vorzugsweise derart gestaltet, dass die Kontur des Innenkörpers 10 möglichst exakt, d. h. ohne viel Bewegungsspielraum, darin aufgenommen ist und parallel zur Richtung der äußeren Schanzschrauben 15, 17 vertikal bewegt werden kann. Der Innenkörper 10 ist vorzugsweise aus dem gleichen Material wie der Grundkörper 3 ausgebildet. Die inneren Schanzschrauben 19 sind an dem Innenkörper 10 in ähnlicher Weise angebracht wie die äußeren Schanzschrauben 15, 17 am Grundkörper 3, d. h. sie sind im funktionalen Zustand relativ zum Innenkörper 10 nicht mehr drehbar und mittels einer Befestigungseinrichtung 23 vertikal bezogen auf den Innenkörper 10 festgelegt.

Eine Verschiebeeinrichtung 21 sorgt für das Zusammenwirken, also das relative Verschieben, des Grundkörpers 3 mit dem Innenkörper 10 und damit den daran angeordneten inneren Schanzschrauben 19. In einer Bohrung , die im Wesentlichen parallel zu den anderen Bohrungen 12 etwa in der Mitte des Grundkörpers 3 angeordnet ist, ist eine Schraube 26 angebracht, die ein Außengewinde aufweist, das mit einer Gewindebohrung etwa in der Mitte des Innenkörpers 10 im Eingriff ist und bei Drehung eine vertikale Verschiebung des Innenkörpers 10 bezüglich des Grundkörpers 3 bewirkt. Bezüglich des Grundkörpers 3 ist die Schraube 26, die integral mit der Verschiebeeinrichtung 21 ausgebildet sein kann, drehbar innerhalb der Bohrung, wobei die Verschiebeeinrichtung 21 durch ein entsprechendes Führungselement derart am Grundkörper 3 angebracht ist, dass die Verschiebeeinrichtung 21 sich bezüglich der Drehachse 20 drehen kann, aber in Vertikalrichtung nicht verschoben werden kann. Eine Drehung des Stellrads der Verschiebeeinrichtung 21, beispielsweise durch eine Bedienperson auf der mit Rändelung 22 versehenen Umfangsfläche bewirkt eine vertikale Verschiebung des Innenkörpers 10 und damit der inneren Schanzschrauben 19. Der maximale Knochendistraktionsweg wird bei dieser Ausführungsform durch den Abstand bestimmt, um den der Innenkörper 10 innerhalb des Aufnahmeraums 36 bewegt werden kann.

Es ist anzumerken, dass die Darstellung der Fig. 9 rein schematisch ist, um das Zusammenspiel der Verschiebeeinrichtung 21 mit Grund- bzw. Innenkörper zu erläutern. Wie oben beschrieben ist hier die Oberseite der Verschiebeeinrichtung 21 derart ausgebildet, dass sie mit der Oberseite 5 des Grundkörpers 3 bündig abschließt, wobei dieser Übergang Kanten- und vorsprungsfrei ausgebildet ist. Da bei der hier dargestellten Ausführungsform die Verschiebeeinrichtung 21 mit einem Stellrad versehen ist, muss die Oberseite 5 des Grundkörpers 3 entsprechend eine Ausnehmung aufweisen und abgeflacht sein, so dass die gesamte Oberfläche der Fixiervorrichtung 1 eine im Wesentlichen ebene, vorsprungsfreie Fläche bildet. Auch in dieser, vierten Ausführungsform kann das Stellrad 21 der Verschiebeeinrichtung auf seiner Umfangsfläche eine Skala bzw. einen Maßstab aufweisen, der dazu dient, die Verschiebung der inneren Schanzschrauben 19 bezüglich des Grundkörpers 3 zu messen und einzustellen.

Alternativ zur Anbringung der Verschiebeeinrichtung 21 auf der Oberseite 5 des Grundkörpers 3 kann die Verschiebeeinrichtung 21 auch innerhalb des Grundkörpers 3 angeordnet sein, wie beispielsweise in der ersten Ausführungsform in Fig. 1 für die Verschiebeeinrichtung der äußeren Schanzschrauben 15, 17 dargestellt.

Weitere mögliche Konstruktionen und Ausführungsformen der erfindungsgemäßen Fixiervorrichtung 1 betreffen die Ausführung des Grundkörpers 3, der beispielsweise als Hohlkörper ausgebildet sein kann. Dabei übernehmen entsprechende Einsätze, die fest mit dem Grundkörper 3 verbunden sind, die Befestigungsaufgaben für die inneren und äußeren Schanzschrauben und die Verschiebeeinrichtung(en).

Mit dem erfindungsgemäßen Gegenstand wird eine Fixiervorrichtung bereitgestellt, die problemlos handhabbar, einfach aufgebaut und leicht ist sowie den täglichen Gebrauch und die Verwendung während einer Operation erleichtert.

### Bezugszeichenliste:

- 1: Fixiervorrichtung
- 3: Grundkörper
- 5: Oberseite
- 7: Unterseite
- 9: Mittelabschnitt
- 10: Innenkörper
- 11: erstes Ende
- 12: Bohrung
- 13: zweites Ende
- 14: Symmetrieachse
- 15: erste äußere Schanzschraube
- 16: Gewinde
- 17: zweite äußere Schanzschraube
- 18: Gewinde
- 19: innere Schanzschraube
- 20: Drehachse
- 21: Verschiebeeinrichtung
- 21a: Stellrad
- 22: Rändelung
- 23: Befestigungseinrichtung
- 24: Verzahnung
- 25: Gewindebohrung
- 26: Schraube
- 27: Führungselement
- 29: Knochengewinde
- 31: Spitze
- 32: Schaft
- 33: Innengewinde
- 34: Schraub- oder Klemmelement
- 36: Aufnahmeraum

## Patentansprüche

1. Fixiervorrichtung (1) zur transversalen Knochendistraktion mit
einem länglichen Grundkörper (3), der eine knochenabgewandte Oberseite (5), eine knochenzugewandte Unterseite (7), einen Mittelabschnitt (9) sowie ein erstes Ende (11) und ein zweites Ende (13) aufweist,
einer ersten äußeren Schanzschraube (15), die an dem ersten Ende (11) auf der Unterseite (7) des Grundkörpers (3) angebracht ist, und einer zweiten äußeren Schanzschraube (17), an dem zweiten Ende (13) auf der Unterseite (7) des Grundkörpers (3) angebracht ist,
mindestens einer inneren Schanzschraube (19), die in dem Mittelabschnitt (9) auf der Unterseite (7) des Grundkörpers (3) angebracht ist, und
mindestens einer Verschiebeeinrichtung (21), die dazu eingerichtet ist, die mindestens eine innere Schanzschraube (19) relativ zu den äußeren Schanzschrauben (15, 17) vom Knochen weg zu verschieben,
wobei die inneren und äußeren Schanzschrauben (15, 17, 19) dazu eingerichtet sind, an ihrem knochenzugewandten Ende mit dem Knochen in Eingriff gebracht zu werden,
**dadurch gekennzeichnet, dass** die mindestens eine innere Schantzschraube (19), die äußeren Schanzschrauben (15, 17) und die Verschiebeeinrichtung (21) in einem funktionalen Zustand der Fixiervorrichtung (1) nicht über die knochenabgewandte Oberseite (5) des Grundkörpers (3) hinausragen, so dass eine knochenabgewandte Oberfläche der Fixiervorrichtung (1) eine im Wesentlichen ebene, vorsprungsfreie Fläche bildet.

2. Fixiervorrichtung (1) nach Anspruch 1, **dadurch gekennzeichnet, dass** bei Betätigung der Verschiebeeinrichtung (21)
a) die mindestens eine innere Schanzschraube (19) mit dem Grundkörper (3) festgelegt ist, oder
b) die mindestens eine innere Schanzschraube (19) relativ zum Grundkörper (3) bewegbar ist.

3. Fixiervorrichtung (1) nach einem der vorhergehenden Ansprüche, **dadurch gekennzeichnet, dass** die Verschiebeeinrichtung (21) mindestens ein Teil aus der Gruppe aufweist, die einen Rändel- oder Drehkopf, einen profilierten Schraubenkopf, eine Rändelmutter, eine Rändelschraube, eine Schraube (26) und ein Führungselement (27) umfasst.

4. Fixiervorrichtung (1) nach einem der vorhergehenden Ansprüche, **dadurch gekennzeichnet, dass** sie mindestens eine Befestigungseinrichtung (23) aufweist, die jeweils dazu eingerichtet ist, eine Verschiebung der inneren bzw. äußeren Schanzschrauben (15, 17, 19) zu verhindern oder zu ermöglichen.

5. Fixiervorrichtung (1) nach Anspruch 4, **dadurch gekennzeichnet, dass** die Befestigungseinrichtung (23) eine Bohrung auf einer Seite des Grundkörpers (3) und ein darin aufgenommenes Befestigungsmittel aufweist.

6. Fixiervorrichtung (1) nach einem der vorhergehenden Ansprüche, **dadurch gekennzeichnet, dass** die inneren und/oder äußeren Schanzschrauben (15, 17, 19) an ihrem oberen Ende ein metrisches Gewinde (16, 18) und an ihrem unteren Ende ein zur Knochenbefestigung geeignetes Gewinde (29) aufweisen.

7. Fixiervorrichtung (1) nach einem der vorhergehenden Ansprüche, **dadurch gekennzeichnet, dass** die Verschiebeeinrichtung (21) ein Stellelement aufweist, das in einer Ausnehmung des Grundkörpers (3) aufgenommen ist, wobei eine Bewegung des Stellelements eine Verschiebung der mindestens einen inneren Schanzschraube (19) relativ zu den äußeren Schanzschrauben (15, 17) bewirkt.

8. Fixiervorrichtung (1) nach einem der vorhergehenden Ansprüche, **dadurch gekennzeichnet, dass** der Grundkörper (3) seitlich mindestens eine Ausnehmung aufweist.

9. Fixiervorrichtung (1) nach einem der vorhergehenden Ansprüche, **dadurch gekennzeichnet, dass** die Verschiebeeinrichtung (21) einen bevorzugt länglichen Innenkörper (10) umfasst, wobei der Innenkörper (10) vorzugsweise in einem Aufnahmeraum auf der Unterseite (7) des Grundkörpers (3) aufnehmbar ist.

10. Fixiervorrichtung (1) nach Anspruch 9, **dadurch gekennzeichnet, dass** die mindestens eine innere Schanzschraube (19) an dem Innenkörper (10) angebracht ist.

11. Fixiervorrichtung (1) nach Anspruch 9 oder 10, **dadurch gekennzeichnet, dass** die Verschiebeeinrichtung (21) eine Gewindeschraube (26) mit einem Kopf umfasst, die den Grundkörper (3) mit dem Innenkörper (10) verbindet und dazu eingerichtet ist, durch Drehen den Innenkörper (10) relativ zum Grundkörper (3) zum Knochen hin oder vom Knochen weg zu bewegen.

12. Fixiervorrichtung (1) nach einem der vorhergehenden Ansprüche, **dadurch gekennzeichnet, dass** der Grundkörper (3) zumindest teilweise als rohrförmiger Hohlkörper ausgebildet ist.

13. Fixiervorrichtung (1) nach einem der vorhergehenden Ansprüche, **dadurch gekennzeichnet, dass** zumindest der Grundkörper (3) mittels 3D-Druck hergestellt ist.

14. Fixiervorrichtung (1) nach einem der vorhergehenden Ansprüche, **dadurch gekennzeichnet, dass** die Verschiebeeinrichtung mittels Aktuator, vorzugsweise elektrisch, betreibbar ist.

## Claims

1. Fixation device (1) for transverse bone distraction, comprising
an elongated base (3) having a bone-remote top surface (5), a bone-facing bottom surface (7), a central section (9), a first end (11) and a second end (13),
a first outer Schanz screw (15) attached to the first end (11) on the bottom surface (7) of the base (3), and a second outer Schanz screw (17) attached to second end (13) on the bottom surface (7) of the base (3),
at least one inner Schanz screw (19) attached to the central section (9) on the bottom surface (7) of the base (3), and
at least one displacement device (21) configured to move the at least one inner Schanz screw (19) away from the bone relative to the outer Schanz screws (15, 17),
the inner and outer Schanz screws (15, 17, 19) being configured to engage with the bone at their bone-facing ends,
**characterized in that**
the at least one inner Schanz screw (19), the outer Schanz screws (15, 17) and the displacement device (21) in a functional state of the fixation device (1) do not protrude beyond the bone-remote top surface of the elongate base (3), such that the a bone-remote surface of the fixation device (1) forms a substantially flat, projectionfree surface.

2. Fixation device (1) according to claim 1, **characterized in that** when the displacement device (21) is actuated,
a) the at least one inner Schanz screw (19) is fixed to the base (3), or
b) the at least one inner Schanz screw (19) is movable relative to the base (3).

3. Fixation device (1) according to one of the preceding claims, **characterized in that** the displacement device (21) comprises at least one component of the group consisting of a knurled or rotating head, a profiled screw head, a knurled nut, a knurled screw, a screw (26) and a guide element (27).

4. Fixation device (1) according to one of the preceding claims, **characterized in that** it comprises at least one fastening device (23), each fastening device (23) being configured to prevent or to enable a displacement of the inner Schanz screws (15, 17) or the outer Schanz screws (19), respectively.

5. Fixation device (1) according to claim 4, **characterized in that** the fastening device (23) comprises a bore on one side of the base (3) and a fastening means accommodated therein.

6. Fixation device (1) according to one of the preceding claims, **characterized in that** the inner and/or outer Schanz screws (15, 17, 19) comprise a metric thread (16, 18) at their upper end and a suitable thread (29) for bone fixation at their lower end.

7. Fixation device (1) according to one of the preceding claims, **characterized in that** the displacement device (21) comprises an adjusting element accommodated in a recess in the base (3) wherein a movement of the adjusting element causes a displacement of the at least one inner Schanz screw (19) relative to the outer Schanz screws (15, 17).

8. Fixation device (1) according to one of the preceding claims, **characterized in that** the base (3) comprises at least one recess on the side.

9. Fixation device (1) according to one of the preceding claims, **characterized in that** the displacement device (21) comprises a preferably elongated inner body (10), the inner body (10) preferably being receivable in a receiving space on the bottom surface (7) of the base (3).

10. Fixation device (1) according to claim 9, **characterized in that** the at least one inner Schanz screw (19) is attached to the inner body (10).

11. Fixation device (1) according to claim 9 or 10, **characterized in that** the displacement device (21) comprises a threaded screw (26) with a head, the threaded screw (26) connecting the base (3) to the inner body (10) and being configured to move the inner body (10) relative to the base (3) towards or away from the bone by rotation.

12. Fixation device (1) according to one of the preceding claims, **characterized in that** the base (3) is at least partially configured as a tubular hollow body.

13. Fixation device (1) according to one of the preceding claims, **characterized in that** at least the base (3) is produced by means of 3D printing.

14. Fixation device (1) according to one of the preceding claims, **characterized in that** the displacement device is operable by means of an actuator, preferably electrically.

## Revendications

1. Dispositif de fixation (1) pour une distraction osseuse transversale comprenant
un corps de base (3) longitudinal qui présente un côté supérieur (5) détourné de l'os, un côté inférieur (7) tourné vers l'os, une section centrale (9) ainsi qu'une première extrémité (11) et une seconde extrémité (13),
une première vis de Schanz extérieure (15) qui est apposée contre la première extrémité (11) sur le côté inférieur (7) du corps de base (3), et une seconde vis de Schanz extérieure (17) qui est apposée contre la seconde extrémité (13) sur le côté inférieur (7) du corps de base (3),
au moins une vis de Schanz intérieure (19) qui est apposée dans la section centrale (9) sur le côté inférieur (7) du corps de base (3), et
au moins un équipement de déplacement (21) qui est aménagé de façon à déplacer en l'éloignant de l'os la au moins une vis de Schanz intérieure (19) par rapport aux vis de Schanz extérieures (15, 17),
dans lequel les vis de Schanz intérieures et extérieures (15, 17, 19) sont aménagées de façon à être amenées en prise avec l'os au niveau de leur extrémité tournée vers l'os,
**caractérisé en ce que** dans un état opérationnel du dispositif de fixation (1) la au moins une vis de Schanz intérieure (19), les vis de Schanz extérieures (15, 17) et l'équipement de déplacement (21) ne dépassent pas par-dessus le côté supérieur (5) détourné de l'os du corps de base (3), de sorte qu'une surface supérieure du dispositif de fixation (1) détournée de l'os forme une surface essentiellement plane et exempte de saillie.

2. Dispositif de fixation (1) selon la revendication 1, **caractérisé en ce que** dans le cas d'un actionnement de l'équipement de déplacement (21)
a) la au moins une vis de Schanz intérieure (19) est fixée avec le corps de base (3), ou
b) la au moins une vis de Schanz intérieure (19) peut être déplacée par rapport au corps de base (3).

3. Dispositif de fixation (1) selon l'une des revendications précédentes, **caractérisé en ce que** l'équipement de déplacement (21) présente au moins une pièce du groupe qui comprend une tête moletée ou rotative, une tête de vis profilée, un écrou moleté, une vis moletée, une vis (26) et un élément de guidage (27).

4. Dispositif de fixation (1) selon l'une des revendications précédentes, **caractérisé en ce qu'**il présente au moins un équipement d'attache (23) qui est respectivement aménagé de façon à empêcher ou permettre un déplacement des vis de Schanz intérieures ou extérieures (15, 17, 19).

5. Dispositif de fixation (1) selon la revendication 4, **caractérisé en ce que** l'équipement d'attache (23) présente un alésage sur un côté du corps de base (3) et un moyen d'attache qui y est reçu.

6. Dispositif de fixation (1) selon l'une des revendications précédentes, **caractérisé en ce que** les vis de Schanz intérieures et/ou extérieures (15, 17, 19) présentent un filetage métrique (16, 18) au niveau de leur extrémité supérieure et un filetage (29) adapté à l'attache osseuse au niveau de leur extrémité inférieure.

7. Dispositif de fixation (1) selon l'une des revendications précédentes, **caractérisé en ce que** l'équipement de déplacement (21) présente un élément de réglage qui est reçu dans un évidement du corps de base (3), dans lequel un mouvement de l'élément de réglage provoque un déplacement de la au moins une vis de Schanz intérieure (19) par rapport aux vis de Schanz extérieures (15, 17).

8. Dispositif de fixation (1) selon l'une des revendications précédentes, **caractérisé en ce que** le corps de base (3) présente latéralement au moins un évidement.

9. Dispositif de fixation (1) selon l'une des revendications précédentes, **caractérisé en ce que** l'équipement de déplacement (21) comprend un corps intérieur (10) de préférence longitudinal, dans lequel le corps intérieur (10) peut de préférence être reçu dans un espace de réception sur le côté inférieur (7) du corps de base (3).

10. Dispositif de fixation (1) selon la revendication 9, **caractérisé en ce que** la au moins une vis de Schanz intérieure (19) est apposée contre le corps intérieur (10).

11. Dispositif de fixation (1) selon la revendication 9 ou 10, **caractérisé en ce que** l'équipement de déplacement (21) comprend une vis filetée (26) avec une tête, laquelle vis filetée relie le corps de base (3) avec le corps intérieur (10) et est aménagée pour déplacer par rotation, en le rapprochant de l'os ou en l'éloignant de l'os, le corps intérieur (10) par rapport au corps de base (3).

12. Dispositif de fixation (1) selon l'une des revendications précédentes, **caractérisé en ce que** le corps de base (3) est conçu au moins partiellement en tant que corps creux tubulaire.

13. Dispositif de fixation (1) selon l'une des revendications précédentes, **caractérisé en ce qu'**au moins le corps de base (3) est fabriqué au moyen d'une impression 3D.

14. Dispositif de fixation (1) selon l'une des revendications précédentes, **caractérisé en ce que** l'équipement de déplacement peut être entraîné au moyen d'un actionneur, de préférence électrique.
